# EUROPÄISCHE PATENTSCHRIFT

(11) **EP 0 841 098 B1**
(45) Veröffentlichungstag und Bekanntmachung des Hinweises auf die Patenterteilung: **28.08.2002**
(21) Anmeldenummer: 97116673.1
(22) Anmeldetag: 24.09.1997
(51) Int. Cl.: B05C 17/01, A61C 9/00, A61C 5/06

(54) **Vorrichtung zum Ausbringen einer fliessfähigen Masse**
Device for discharging a fluent mass
Dispositif de décharge d'une masse fluide

(30) Priorität: 11.11.1996 DE 29619558 U
(43) Veröffentlichungstag der Anmeldung: 13.05.1998
(73) Patentinhaber: 3M ESPE AG, 82229 Seefeld (DE)
(72) Erfinder: Hammen, Klaus, 82205 Gilching (DE); Brandhorst, Gerd, 86899 Landsberg (DE)
(74) Vertreter: Strehl Schübel-Hopf & Partner

(56) Entgegenhaltungen:
- US-A- 4 330 280
- US-A- 5 022 563
- US-A- 5 129 825

## Beschreibung

Aus DE-U-29 502 783 ist ein Behälter zum Lagern und Ausbringen einer fließfähigen Masse, insbesondere einer Dentalmasse bekannt, der eine Kartusche zur Aufnahme der Masse mit einer an seinem vorderen Ende ansetzenden Ausbringtülle in Form eines gekrümmten Röhrchens und einem an seinem hinteren Ende vorgesehenen Flansch zum Einsetzen in einen Applikator aufweist. Der Flansch hat vorzugsweise die Form eines sechseckigen Prismas, wodurch gemäß der Beschreibung in DE-U-29 502 783 erreicht werden soll, daß beim Einlegen der Kartusche in den Applikator das Ausbringende der Tülle diesem gegenüber seine Orientierung beibehält. Dies ist insbesondere dann von Bedeutung, wenn kleine Mengen der in der Kartusche enthaltenen Masse an örtlich begrenzten Stellen genau auszubringen sind, wie es bei Dentalmassen der Fall ist, die direkt auf eine Behandlungsstelle aufgetragen, etwa in eine Zahnkavität gefüllt, werden sollen.

Eine Ausbringvorrichtung mit den im ersten Teil des Anspruchs 1 angegebenen Merkmalen ist aus US-A-5,129,825 bekannt. Bei einer der dort beschriebenen Ausführungen hat das hintere Kartuschenende die Form eines Sterns, dessen Nuten mit einem Vorsprung im Applikator so zusammenwirken, daß bei Drehung der Kartusche die Nuten eine Ratschenbewegung relativ zu dem Vorsprung ausführen und dadurch die gewünschte Winkeljustierung bestimmen. Bei einer anderen, in derselben Druckschrift beschriebenen Ausführung ist das hintere Kartuschenende glatt und läßt sich quer zur Achse in eine ebenfalls glatte Aufnahme des Applikators einsetzen.

Die Erfindung betrifft eine Weiterbildung der bekannten Anordnung mit der Aufgabe, deren Handhabbarkeit weiter zu verbessern.

Die erfindungsgemäße Lösung dieser Aufgabe ist im Anspruch 1 gekennzeichnet. Danach wird erreicht, daß sich zwar wie beim Stand der Technik die Kartusche bei Benutzung gegenüber dem Applikator nicht unbeabsichtigt verdreht, dennoch eine bewußte Änderung der relativen Stellung von Kartusche und Applikator und damit eine Änderung des Ausbringwinkels während der Arbeit mit geringem Kraftaufwand zuläßt, ohne die Kartusche aus dem Applikator herausnehmen und in anderer Orientierung erneut einlegen zu müssen. Dabei ergibt die Polygonform sowohl einen ausreichenden Durchmesserunterschied zwischen gegenüberliegenden Ecken einerseits und gegenüberliegenden Seiten andererseits und damit eine ausgeprägte Rastung als auch eine Veränderung der Drehstellung in brauchbar kleinen Schritten ergibt. Trotz dieser Rastung läßt sich die Vorrichtung vom Benutzer in einfacher Weise zusammensetzen.

Die Weiterbildung nach den Ansprüchen 3 bis 5 ist vom Standpunkt der Fertigung der erfindungsgemäßen Vorrichtung günstig.

Die Ansprüche 2 und 6 beziehen sich auf für die praktische Handhabung besonders geeignete Gestaltungen.

Ein Ausführungsbeispiel der Erfindung wird nachstehend anhand der Zeichnungen näher erläutert. Darin zeigt
- Figur 1: einen Applikator mit in Axialrichtung geschnittenem Griffteil, und
- Figur 2: den Applikator mit eingelegter Kartusche.

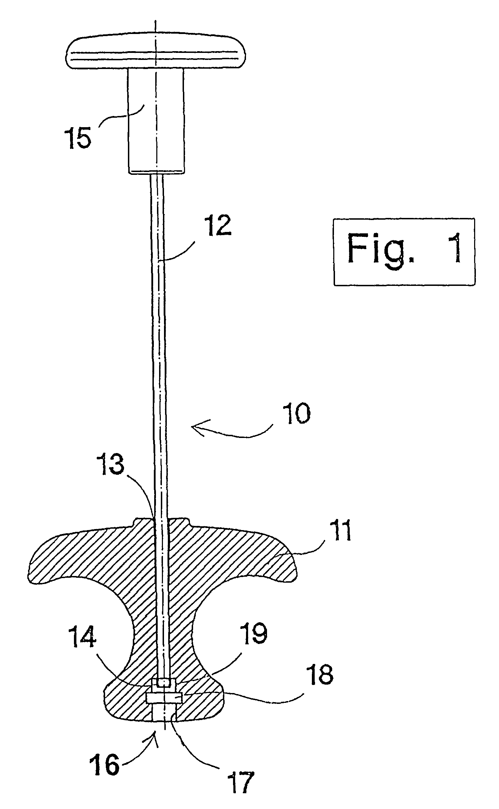

Gemäß Figur 1 besteht der Applikator **10** aus einem Griffteil **11** und einer Kolbenstange **12**. Die Kolbenstange **12** durchsetzt eine Führungsbohrung **13** des Griffteils **11**, weist an ihrem vorderen (in Figur 1 unteren) einen Arretierteil **14** auf und trägt an ihrem hinteren Ende ein Daumen-Druckelement **15**.

In dem Griffteil **11** ist eine Schiebeführung **16** ausgebildet, die einen äußeren Aufnahmeabschnitt **17,** einen mittleren Aufnahmeabschnitt **18** und einen inneren Abschnitt **19** aufweist. Die drei Abschnitte **17, 18** und **19** sind quer zur Achse (in Figur 1 zum Betrachter hin) offen und weisen jeweils einen halb-zylindrischen Boden mit zwei daran anschließenden, gegenüberliegenden und zueinander parallelen Wandabschnitten auf. Der mittlere Abschnitt **18** hat einen größeren Durchmesser als der äußere und der innere Abschnitt **17, 19**.

Bei dem Arretierteil **14** handelt es sich um das vordere Ende der Kolbenstange **12**, das durch Quetschung in seinem Querschnitt gegenüber dem der Führungsbohrung **13** so vergrößert ist, daß die Kolbenstange **12** nicht nach hinten aus dem Griffteil **11** herausgezogen werden kann. Die axiale Länge des Arretierteils **14** ist etwas kürzer als die axiale Länge des inneren Abschnitts **19** der Schiebeführung **16**.

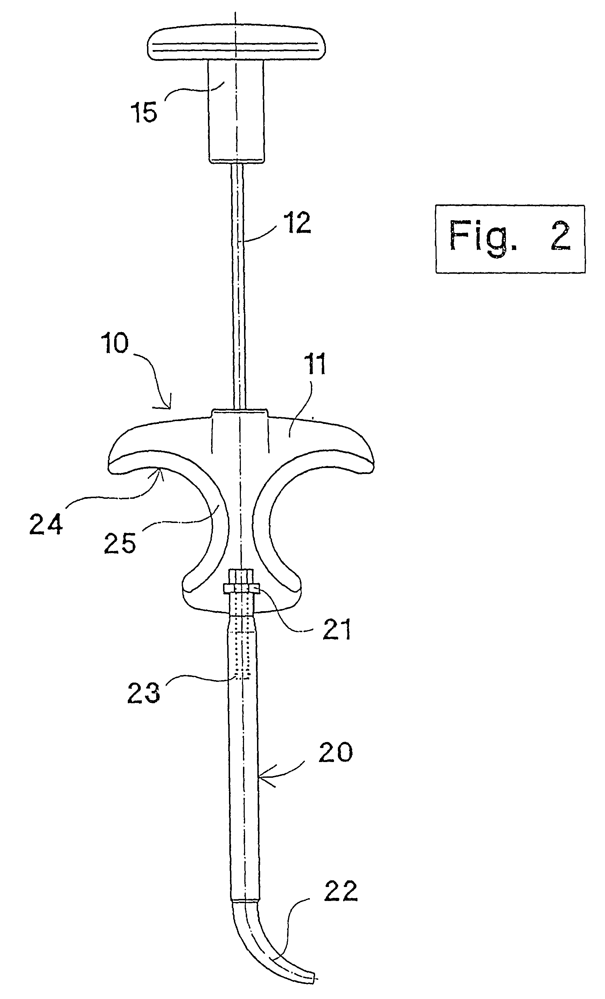

In Figur 1 ist die Kolbenstange **12** in der Stellung gezeigt, in dem sie gegenüber dem Griffteil **11** so weit wie möglich zurückgezogen ist. In dieser Stellung liegen die Abschnitte **17** und **18** der Schiebeführung **16** frei. In diese Abschnitte läßt sich eine Kartusche **20** einlegen, wobei ein am hinteren Kartuschenende vorgesehener Flansch **21** in den mittleren Abschnitt **18** der Schiebeführung **16** eingreift. Dieser Zustand ist in Figur 2 gezeigt.

Der Flansch **21** hat die Form eines regelmäßig achteckigen Prismas, wobei das Durchmessermaß zwischen zwei gegenüberliegenden Flächen des Prismas etwas kleiner ist als der Abstand zwischen den gegenüberliegen, zueinander parallelen Wandabschnitten des mittleren Abschnitts **18** der Schiebeführung **16**. Da auch das hintere, zylindrische Ende der Kartusche **20** einen etwas geringeren Durchmesser hat als der äußere Aufnahmeabschnitt **17**, läßt sich die Kartusche **20** problemlos in seitlicher Richtung in die Schiebeführung **16** einlegen und in die in Figur 2 gezeigte Position bringen.

Das Durchmessermaß zwischen zwei gegenüberliegenden Kanten des achteckig-prismatischen Flansches **21** ist geringfügig größer als der genannte Abstand zwischen den gegenüberliegen Wandabschnitten des mittleren Abschnitts **18** der Schiebeführung **16.** Das Material der Kartusche **20** und des daran angeformten Flansches **21** ist elastisch verformbar, so daß sich die Kartusche **20** unter Aufwendung einer mäßigenden Kraft um ihre Längsachse gegenüber dem Griffteil **11** des Applikators **10** drehen läßt. Zusätzlich oder alternativ kann die gewünschte Elastizität auch durch entsprechende Wahl des Materials des Griffteils **11** erzeugt werden.

Zum Aufbringen einer solchen Kraft kann die Kartusche **20** an einer an ihrem vorderen Ende ansetzenden gekrümmten Ausbringtülle **22** erfaßt werden. Beim Drehen wird eine Position überwunden, in der zwei Kanten des achteckig-prismatischen Flansches **21** den parallelen Wänden des mittleren Abschnitts **18** der Schiebeführung **16** gegenüberstehen. Beim weiteren Drehen rastet die Kartusche in eine gegenüber der Ausgangsstellung um **45°** verdrehte Stellung, in der wiederum zwei Flächen des Flansches **21** den Wänden des mittleren Abschnitts **18** der Schiebeführung **16** gegenüberstehen.

Wichtig ist, daß sich die beschriebene Drehung durchführen läßt, während die Kolbenstange **12** in die Kartusche **20** eingreift und mit ihrem Arretierteil **14** (dessen Durchmessermaß kleiner ist als der Innendurchmesser der Kartusche **20**) am hinteren Ende eines in der Kartusche **20** vorhandenen Ausbringkolbens **23** anliegt.

Somit ist es, um während der Benutzung die Drehstellung zwischen Applikator **10** und Kartusche **20** zu ändern, nicht erforderlich, die Kolbenstange **12** zurückzuziehen, die Kartusche **20** aus der Schiebeführung **16** herauszunehmen und in anderer Orientierung wieder einzusetzen. Vielmehr läßt sich die Drehstellung unmittelbar vor dem Beginn des Ausbringvorgangs - oder sogar während desselben - verändern, wenn das Gerät bereits in seine endgültige Stellung geführt wurde und das Ende der Ausbringtülle **22** sich an der Behandlungsstelle befindet.

Der Griffteil **11** ist mit zwei gegenüberliegenden Finger-Halteteilen **24** versehen, die zur besseren Anlage an Zeigeund Mittelfinger mit verbreiterten Anlageflanschen **25** versehen sind. Der Applikator **10** läßt sich daher mit einer Hand bedienen, so daß die andere Hand bei Bedarf zum Drehen der Kartusche **20** gegenüber dem Griffteil **11** zur Verfügung steht.

Bei Dentalmassen, etwa Zahnfüllmassen zum direkten Ausbringen in eine Zahnkavität, hat die Kartusche **20** einen Innendurchmesser von beispielsweise 1,5 bis 5 mm, vorzugsweise ca. 2,5 mm und das Austrittsende der sich verjüngenden gekrümmten Ausbringtülle **22** einen Innendurchmesser von beispielsweise 1,5 bis 3 mm, vorzugsweise etwa 2 mm. Bei dem Griffteil **11** und dem Daumen-Druckelement **15** handelt es sich um Kunststoff-Spritzteile. Die Kolbenstange **12** besteht vorzugsweise aus einem Federstahldraht, der mit dem Daumen-Druckelement **15** umspritzt ist.

## Patentansprüche

1. Vorrichtung zum Ausbringen einer fließfähigen Masse, insbesondere einer Dentalmasse, umfassend
eine Kartusche (**20**) zur Aufnahme der Masse mit einer an ihrem vorderen Ende ansetzenden Ausbringtülle (**22**), deren Ausbringende unter einem Winkel zur Kartuschenachse verläuft, und einem an ihrem hinteren Ende vorgesehenen prismatischen Flansch (**21**), und
einen Applikator (**10**) mit einer Kolbenstange (**12**) zum Vorschub eines in der Kartusche (**20**) angeordneten Ausbringkolbens (**23**) und einer Aufnahme (**16**) für den Flansch (**21**), der einen von der Kreisform abweichenden Querschnitt aufweist,
wobei das Material des Flansches (**21**) und/oder der Aufnahme (**16**) derart elastisch ist, daß es eine rastende Drehung der Kartusche (**20**) relativ zu dem Applikator (**10**) gestattet,
**dadurch gekennzeichnet,**
**daß** der Flansch (**21**) im Querschnitt die Form eines regelmäßigen Polygons hat, und
**daß** die Aufnahme als quer zur Achse der Kolbenstange (**12**) offene Schiebeführung (**16**) ausgebildet ist und ihr von zwei gegenüberliegenden parallelen Wänden der Schiebeführung bestimmtes kleinstes Durchmessermaß zwischen dem kleinsten und dem größten Durchmessermaß des prismatischen Flansches (**21**) liegt.

2. Vorrichtung nach Anspruch 1, wobei der Flansch (**21**) im Querschnitt die Form eines Achtecks hat.

3. Vorrichtung nach Anspruch 1 oder 2, wobei die Kolbenstange (**12**) eine in einem Griffteil (**11**) des Applikators (**10**) vorhandene Führungsbohrung (**13**) durchsetzt und an ihrem dem Ausbringkolben (**23**) zugewandten Ende ein Arretierteil (**14**) aufweist, dessen Außenmaß größer ist als der Querschnitt der Führungsbohrung (**13**).

4. Vorrichtung nach Anspruch 3, wobei das Arretierteil (**14**) vom gequetschten vorderen Ende der Kolbenstange (**12**) gebildet ist.

5. Vorrichtung nach Anspruch 3 oder 4, wobei das Griffteil (**11**) einen an die Aufnahme (**16**) anschließenden, gegenüber der Führungsbohrung (**13**) erweiterten Bereich (**19**) zur Aufnahme des Arretierteils (**14**) aufweist.

6. Vorrichtung nach einem der vorhergehenden Ansprüche, wobei die Kolbenstange (**12**) an ihrem von dem Ausbringkolben (**23**) abgewandten hinteren Ende ein Daumen-Druckelement (**15**) trägt und der Griffteil (**11**) mit zwei seitlichen Finger-Halteteilen (**24**) versehen ist.

## Claims

1. A device for dispensing a flowable, particularly dental, substance, comprising
a cartridge (**20**) for receiving the substance, having a dispensing nozzle (**22**) connected to the front end of the cartridge and a dispensing end extending at an angle with respect to the cartridge axis, and a prismatic flange (**21**) provided at the rear end of the cartridge, and
an applicator (**10**) including a piston rod (**12**) for advancing a dispensing piston (**23**) provided inside the cartridge (**20**) and a receiving portion (**16**) for receiving the flange (**21**), which has a cross-sectional shape other than circular,
wherein the material of the flange (**21**) and/or of the receiving portion (**16**) is resilient so as to permit stepwise rotation of the cartridge (**20**) relative to the applicator (**10**),
**characterised in**
**that** the cross-section of the flange (**21**) has the shape of a regular polygon, and
**that** the receiving portion is formed as a guiding slide (**16**) which is open transversely of the axis of the piston rod (**12**), with the smallest diametric dimension of the slide defined by a pair of opposite parallel walls being between the smallest and the largest diametric dimension of the prismatic flange (**21**).

2. The device of claim 1, wherein the cross-section of the flange (**21**) has the shape of an octagon.

3. The device of claim 1 or 2, wherein the piston rod (**12**) extends through a guide bore (**13**) provided in a handle part (**11**) of the applicator (**10**), the end of the piston rod adjacent the dispensing piston (**23**) having a stop member (**14**) the outer dimension of which is larger than the diameter of the guide bore (**13**).

4. The device of claim 3, wherein the stop member (**14**) is formed by the stamp-deformed front end of the piston rod (**12**).

5. The device of claim 3 or 4, wherein the handle part (**11**) has a part (**19**) connected to the receiving portion (**16**) and being larger than the guide bore (**13**), for receiving the stop member (**14**).

6. The device of any preceding claim, wherein the rear end of the piston rod (**12**) remote from the dispensing piston (**23**) carries a thumb-pushing member (**15**), and the handle part (**11**) is provided with two lateral finger holds (**24**).

## Revendications

1. Dispositif de distribution d'une pâte coulante, en particulier d'une pâte dentaire, comprenant :
une cartouche (20) destinée à recevoir la pâte avec un embout de distribution (22) s'appliquant à son extrémité avant et dont l'extrémité de distribution s'étend suivant un angle par rapport à l'axe de la cartouche, et avec une bride (21) prismatique prévue à son extrémité arrière, et
un applicateur (10) avec une tige de piston (12) pour l'avance d'un piston de distribution (23) disposé dans la cartouche (20), et avec un logement (16) pour la bride (21) qui présente une section différente de la forme circulaire,
la matière de la bride (21) et/ou du logement (16) étant élastique au point de permettre une rotation d'encliquetage de la cartouche (20) par rapport à l'applicateur (10),
**caractérisé en ce que**
la bride (21) présente, en section, la forme d'un polygone régulier, et
le logement est réalisé sous la forme d'un guide de coulissement (16) ouvert perpendiculairement à l'axe de la tige de piston, et son plus petit diamètre, déterminé par deux parois opposées et parallèles du guide de coulissement, se situe entre le plus petit et le plus grand diamètre de la bride prismatique (21).

2. Dispositif selon la revendication 1, dans lequel la bride (21) présente, en section, la forme d'un octogone.

3. Dispositif selon la revendication 1 ou 2, dans lequel la tige de piston (12) traverse un trou de guidage (13) ménagé dans une partie formant poignée (11) de l'applicateur (10), et comporte, à son extrémité tournée vers le piston de distribution (23), un élément de blocage (14) dont la cote extérieure est supérieure à la section du trou de guidage (13).

4. Dispositif selon la revendication 3, dans lequel l'élément de blocage (14) est formé par l'extrémité avant écrasée de la tige de piston (12).

5. Dispositif selon la revendication 3 ou 4, dans lequel la partie formant poignée (11) présente une zone (19) se raccordant au logement (16) et élargie par rapport au trou de guidage (13), qui sert à recevoir l'élément de blocage (14).

6. Dispositif selon l'une des revendications précédentes, dans lequel la tige de piston (12) porte, à son extrémité arrière tournée à l'opposé du piston de distribution (23), un élément poussoir pour pouce (15), et la partie formant poignée (11) est pourvue de deux éléments de maintien pour doigts (24) latéraux.
